# FASCICULE DE BREVET EUROPEEN

(11) **EP 3 140 287 B1**
(45) Date de publication et mention de la délivrance du brevet: **07.03.2018**
(21) Numéro de dépôt: 15725827.8
(22) Date de dépôt: 06.05.2015
(51) Int. Cl.: C07D 223/16

(54) **NOUVEAU SEL DE L'IVABRADINE ET SON PROCÉDÉ DE PRÉPARATION**
NEUER IVABRADIN-SALZ UND SEIN HERSTELLUNGSVERFAHREN
NEW IVABRADINE SALT AND ITS PREPARATION PROCESS

(30) Priorité: 06.05.2014 FR 1454081
(43) Date de publication de la demande: 15.03.2017
(73) Titulaire: Les Laboratoires Servier, 92284 Suresnes (FR)
(72) Inventeur: LYNCH, Michael, F-45140 Saint Jean de la Ruelle (FR); COINTEPAS, Patrick, F-45140 Saint Jean de la Ruelle (FR); LAFARGUE, David, F-45650 Saint Jean le Blanc (FR); BRIAULT, Gilles, F-45480 Saint Peravy Epreux (FR)
(86) Numéro de dépôt international: PCT/FR2015/051201
(87) Numéro de publication internationale: WO 2015/170053

(56) Documents cités:
- EP-A1- 0 534 859
- WO-A2-2011/104723
- WO-A2-2011/157720

## Description

La présente invention concerne un nouveau sel de l'ivabradine, son procédé de préparation ainsi que les compositions pharmaceutiques qui le contiennent.

L'ivabradine, ou 3-{3-[{[(7*S*)-3,4-diméthoxybicyclo[4.2.0]octa-1,3,5-trién-7-yl]méthyl}(méthyl)amino]propyl}-7,8-diméthoxy-1,3,4,5-tétrahydro-2*H*-3-benzazépin-2-one, ainsi que ses sels d'addition à un acide pharmaceutiquement acceptable, et plus particulièrement son chlorhydrate, ont des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque, tant systolique que diastolique.

La préparation et l'utilisation en thérapeutique de l'ivabradine et de ses sels d'addition à un acide pharmaceutiquement acceptable, et plus particulièrement de son chlorhydrate, ont été décrites dans le brevet européen EP 0 534 859.

La présente invention concerne l'hémipamoate d'ivabradine de formule (I) : 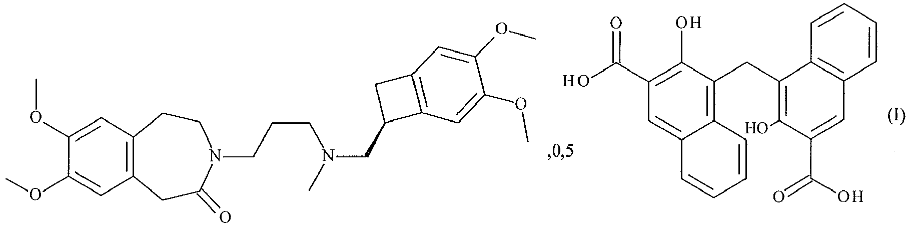 ainsi que ses hydrates, un procédé de préparation dudit sel, et les compositions pharmaceutiques le contenant, en particulier celles permettant une libération contrôlée dans le temps du principe actif.

L'acide pamoïque est aussi nommé acide 4,4'-méthanediylbis(3-hydroxynaphtalène-2-carboxylique).

Le composé de formule (I) présente un ratio ivabradine/acide pamoïque de 1/0,5.

Le composé de formule (I) est obtenu par action du sel disodique de l'acide pamoïque ou pamoate de sodium sur le chlorhydrate d'ivabradine en milieu aqueux.

Le chlorhydrate d'ivabradine et le pamoate de sodium sont mis en présence dans une proportion comprise entre 1/0,5 et 1/0,6.

Le composé de formule (I) ainsi préparé est ensuite extrait du milieu aqueux grâce à un solvant organique, par exemple du dichlorométhane.

Après sa formation, le composé de formule (I) peut être avantageusement repris dans du méthanol afin d'éliminer le solvant organique résiduel.

La présente invention a également pour objet les compositions pharmaceutiques contenant comme principe actif l'hémipamoate d'ivabradine, en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales.

Outre l'hémipamoate d'ivabradine, les compositions pharmaceutiques selon l'invention contiennent un ou plusieurs excipients ou véhicules tels que des diluants, des lubrifiants, des liants, des agents de désintégration, des absorbants, des colorants, des édulcorants.

A titre d'exemple d'excipients ou véhicules, on peut citer :
◆ *pour les diluants* : le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la maltodextrine, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

Le pourcentage d'hémipamoate d'ivabradine dans la composition pharmaceutique est préférentiellement compris entre 5% et 50% en poids.

La posologie utile varie selon le sexe, l'âge et le poids du patient, la voie d'administration, la nature de l'affection et des traitements éventuellement associés et s'échelonne de 2,5 à 30 mg d'ivabradine par 24 heures et plus préférentiellement de 5 à 15 mg par jour et de manière encore préférée de 10 à 15 mg par jour.

Le pourcentage de diluants dans la composition pharmaceutique est préférentiellement compris entre 40% et 80% en poids.

Le pourcentage de lubrifiants dans la composition pharmaceutique est préférentiellement compris entre 0,2% et 10% en poids.

Le pourcentage de liants dans la composition pharmaceutique est préférentiellement compris entre 5% et 50% en poids.

La Demanderesse a trouvé que l'utilisation de l'hémipamoate d'ivabradine permettait la préparation d'une composition pharmaceutique à libération contrôlée du principe actif en s'affranchissant des problèmes générés par les méthodes usuelles.

De nombreuses compositions pharmaceutiques destinées à la libération contrôlée de principes actifs pharmaceutiques ont été proposées et réalisées, pour leur administration par voie orale, buccale, sublinguale, oculaire, rectale, vaginale et/ou parentérale. Ces nouvelles compositions avaient essentiellement pour objectifs :
- de réduire la fréquence d'administration des médicaments,
- d'obtenir des taux relativement constants de principe actif dans le milieu ou au site biologique visé,
- d'obtenir des profils de libération en corrélation avec l'activité pharmacologique des médicaments.

Pour contrôler cette libération, le principe le plus communément employé est d'incorporer le ou les principes actifs avec des excipients, le plus souvent de nature polymérique, dans des matrices.

Quelles que soient les compositions matricielles envisagées, leur obtention se heurte à des problèmes spécifiques de fabrication comme :
- procédé de fabrication complexe et en plusieurs étapes,
- stabilité du principe actif au cours du procédé de fabrication et vis-à-vis des excipients utilisés,
- modulation de la vitesse de libération, du ou des principes actifs, délicate, souvent variable dans le temps et dépendante par exemple de la granulométrie des lots de polymères avec les procédés de compression,
- procédé de fabrication permettant l'obtention d'une formé pharmaceutique essentiellement adaptée à une seule voie d'administration,
- reproductibilité des lots du fait de la multiplication des étapes.

L'utilisation de l'hémipamoate d'ivabradine permet d'obtenir un profil à libération contrôlée du principe actif sans mettre en oeuvre les techniques de formulation galénique complexes décrites dans l'art antérieur.

Ainsi, la Demanderesse a démontré que l'utilisation de l'hémipamoate d'ivabradine dans la composition pharmaceutique de l'invention permettait une libération contrôlée de l'ivabradine, alors même que la formulation galénique utilisée correspond à celle mise en oeuvre avec le chlorhydrate d'ivabradine pour la libération immédiate du principe actif.

Alors que tout le chlorhydrate d'ivabradine est libéré en 15 minutes *in vitro,* le test de dissolution *in vitro* décrit dans la présente demande a montré que seulement 80% de l'hémipamoate d'ivabradine était libéré après 6 heures environ.

Les exemples suivants illustrent l'invention.

### EXEMPLE 1: Préparation de l'hémipamoate d'ivabradine

L'analyse élémentaire a été effectuée sur un appareil Carlo Erba 1108.

Les résultats sont corrigés par la teneur en eau du produit qui est de 1,82% (mesurée par coulométrie).

4,1 g de chlorhydrate d'ivabradine (8,12 mmol) sont solubilisés dans 200 mL d'eau et 2,0 g de pamoate de sodium (4,63 mmol) sont solubilisés dans 200 mL d'eau.

La solution de pamoate de sodium est ajoutée à la solution de chlorhydrate d'ivabradine sous forte agitation. Le sel d'hémipamoate est formé instantanément par précipitation.

L'agitation est maintenue environ 30 minutes, puis l'hémipamoate d'ivabradine est extrait une première fois par 200 mL de dichlorométhane, puis une seconde fois par 100 mL de dichlorométhane. Les fractions organiques sont combinées et rincées avec 100 mL d'eau. La phase organique est séchée avec du sulfate de magnésium donnant une solution limpide de couleur jaune.

La phase organique est évaporée à sec à 40°C sous vide dans un évaporateur rotatif. Une poudre jaune est obtenue.

La poudre jaune est séchée à 40°C sous vide (10 mbar) pendant 16 heures puis reprise dans 200 mL de méthanol.

La solution est évaporée à sec à 40°C sous vide dans un évaporateur rotatif. Une poudre jaune est à nouveau obtenue.

La poudre jaune est séchée à 40°C sous vide (10 mbar) pendant 20 heures.

Le spectre ¹H RMN indique une teneur résiduelle en méthanol de 0,8%.

Le séchage ultérieur de la poudre à 80°C sous vide (10 mbar) pendant 24 heures permet d'obtenir 3,53 g d'un produit présentant une teneur résiduelle en méthanol inférieure à 0,1%.
Rendement = 66,5%

**Analyse élémentaire :**

| Elément | %théorique | % moyen corrigé |
|---|---|---|
| C | 69.77 | 69.40 |
| H | 6.69 | 6.60 |
| N | 4.23 | 4.25 |
| O | 19.31 | |

Méthode de correction des résultats :
Exemple pour C : 69,14*100/(100-1,82) = 69,40%
Exemple pour H : 6,68*100/(100-1.82) - 2*1,82/18 = 6,60% car (il faut tenir compte des atomes d'hydrogène de l'eau (2/18))

### EXEMPLE 2 : Composition pharmaceutique

Formule de préparation pour des comprimés dosés à 5 mg d'ivabradine dans 100 mg

| | |
|---|---|
| Hémipamoate d'ivabradine | 7,07 mg |
| Lactose monohydraté | 62,23 mg |
| Amidon de maïs | 20 mg |
| Maltodextrine (Lycatab® DSH) | 10 mg |
| Stéarate de Magnésium | 0,5 mg |
| Silice colloïdale anhydre (Aérosil 200) | 0,2 mg |

### EXEMPLE 3 : Test de dissolution

### Conditions opératoires de dissolution

Appareil de dissolution à palette décrit à la Pharmacopée Européenne (2.9.3)
Milieu de dissolution : Acide chlorhydrique 0.01 N (pH ∼ 2.1) dégazé
Température du milieu : 37 °C ± 0.5 °C
Volume de milieu : 500 mL ± 5 mL
Vitesse de rotation des pales : 50 t/min ± 2 t/min
Temps de prélèvement standard : 0, 15, 30 et 45 min
Temps de prélèvement ajoutés : 1, 2, 4 6,8, 12 et 16h
Volume prélevé : 1 mL
Remplacement du volume prélevé : non
Nombre d'unités testées : 6
Nombre d'unité par ballon : 1

**Table 1 - Résultats de dissolution**

| | % de principe actif libéré | | | | | | | | |
|---|---|---|---|---|---|---|---|---|---|
| Temps (heure) | B1 | B2 | B3 | B4 | B5 | B6 | Moyenne | Ecart-type | Dérivée |
| 0 | 0 | 0 | 0 | 0 | 0 | 0 | 0 | 0 | 0 |
| 0.25 | 12 | 11 | 12 | 13 | 12 | 11 | 12 | 0.84 | 0.79 |
| 0.5 | 18 | 17 | 18 | 20 | 17 | 16 | 18 | 1.15 | 0.4 |
| 0.75 | 21 | 22 | 22 | 24 | 21 | 20 | 22 | 1.5 | 0.26 |
| 1 | 24 | 25 | 26 | 28 | 23 | 23 | 25 | 1.8 | 0.2 |
| 2 | 38 | 40 | 40 | 43 | 38 | 34 | 39 | 2.87 | 0.23 |
| 4 | 64 | 70 | 71 | 70 | 72 | 66 | 69 | 3 | 0.25 |
| 6 | 81 | 89 | 88 | 85 | 91 | 84 | 86 | 3.58 | 0.15 |
| 8 | 89 | 99 | 96 | 94 | 101 | 93 | 95 | 4.34 | 0.07 |
| 12 | 96 | 109 | 102 | 103 | 110 | 101 | 104 | 5.16 | 0.03 |
| 16 | 98 | 114 | 104 | 106 | 114 | 105 | 107 | 6.27 | 0.01 |

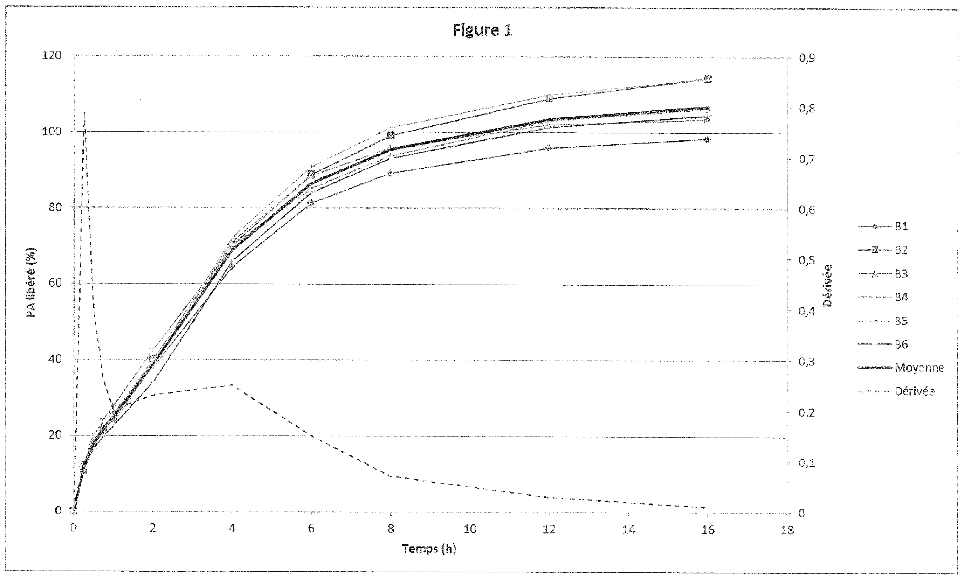

La figure 1 donne une illustration graphique des données présentées dans le tableau ci-dessus.

## Revendications

1. Hémipamoate d'ivabradine de formule (I) : 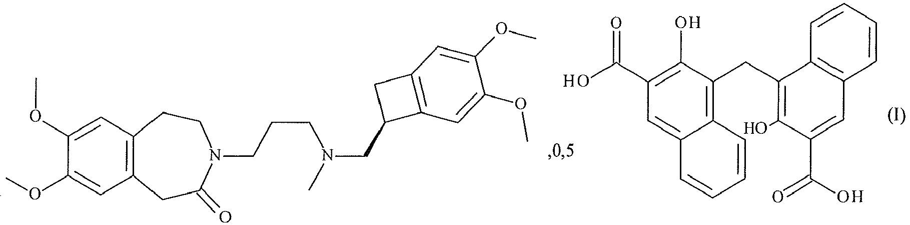 et ses hydrates.

2. Procédé de préparation de l'hémipamoate d'ivabradine, **caractérisé en ce que** le chlorhydrate d'ivabradine est mis en présence du pamoate de sodium en milieu aqueux dans une proportion chlorhydrate d'ivabradine/ pamoate de sodium comprise entre 1/0,5 et 1/0,6.

3. Composition pharmaceutique contenant comme principe actif l'hémipamoate d'ivabradine, en combinaison avec un ou plusieurs excipients ou véhicules inertes, non toxiques, pharmaceutiquement acceptables.

4. Composition pharmaceutique selon la revendication 3, destinée à être utilisée dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque systolique ou diastolique.

## Patentansprüche

1. Ivabradin-Hemipamoat der Formel (I): 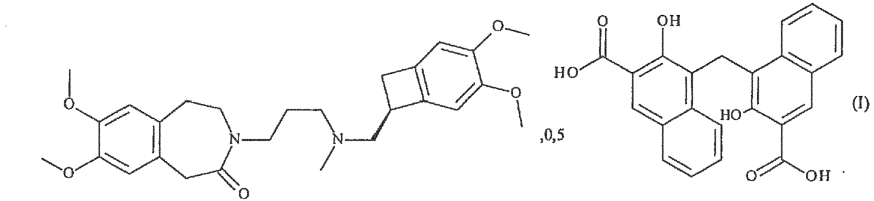 und seine Hydrate.

2. Verfahren zur Herstellung von Ivabradin-Hemipamoat, **dadurch gekennzeichnet, dass** Ivabradin-Hydrochlorid in wässrigem Medium mit Natriumpamoat in einem Verhältnis von Ivabradin-Hydrochlorid/Natriumpamoat zwischen 1/0,5 und 1/0,6 in Kontakt gebracht wird.

3. Pharmazeutische Zubereitung enthaltend als Wirkstoff Ivabradin-Hemipamoat in Kombination mit einem oder mehreren inerten, nichttoxischen und pharmazeutisch annehmbaren Hilfsstoffen oder Trägermaterialien.

4. Pharmazeutische Zubereitung nach Anspruch 3 zur Verwendung bei der Behandlung oder Vorbeugung von verschiedenen klinischen Situationen von Myokardischämie, wie Angina pectoris, Myokardinfarkt und damit verknüpften Rhythmusstörungen sowie bei verschiedenen Erkrankungen mit Rhythmusstörungen, insbesondere supra-ventrikulären, und bei systolischer oder diastolischer Herzinsuffizienz.

## Claims

1. Ivabradine hemipamoate of formula (I): 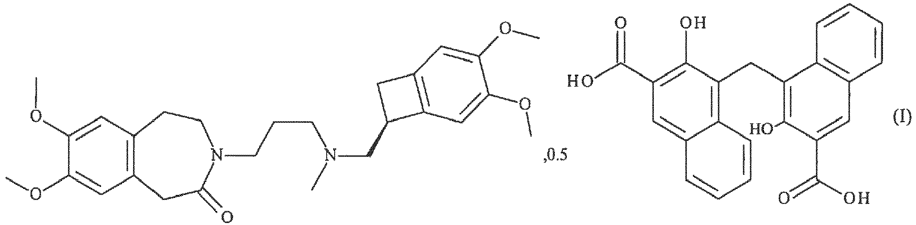 and its hydrates.

2. Process for the preparation of ivabradine hemipamoate, **characterised in that** ivabradine hydrochloride is brought together with sodium pamoate in an aqueous medium in a proportion ivabradine hydrochloride/sodium pamoate of from 1/0.5 to 1/0.6.

3. Pharmaceutical composition comprising as active ingredient ivabradine hemipamoate, in combination with one or more inert, non-toxic, pharmaceutically acceptable excipients or carriers.

4. Pharmaceutical composition according to claim 3 for use in the treatment or prevention of the various clinical situations of myocardial ischaemia such as angina pectoris, myocardial infarction and the associated rhythm disturbances, as well as in the various pathologies involving rhythm disturbances, especially supraventricular rhythm disturbances, and in systolic or diastolic heart failure.
